(19) Europäisches Patentamt / European Patent Office / Office européen des brevets

(11) **EP 4 613 797 A1**

(12) **EUROPEAN PATENT APPLICATION**
published in accordance with Art. 153(4) EPC

(43) Date of publication:
**10.09.2025 Bulletin 2025/37**

(21) Application number: **23885770.0**

(22) Date of filing: **31.10.2023**

(51) International Patent Classification (IPC):
*C08J 3/12* (2006.01)        *A61K 8/73* (2006.01)
*C08B 11/04* (2006.01)       *C08B 37/08* (2006.01)

(52) Cooperative Patent Classification (CPC):
**A61K 8/73; C08B 11/04; C08B 37/003; C08J 3/12**

(86) International application number:
**PCT/JP2023/039305**

(87) International publication number:
**WO 2024/096016 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.10.2022 PCT/JP2022/040829**

(71) Applicant: **Soken Chemical & Engineering Co., Ltd.**
**Toshima-ku**
**Tokyo 171-8531 (JP)**

(72) Inventor: **KOJIMA, Ryota**
**Tokyo 171-8531 (JP)**

(74) Representative: **Gulde & Partner**
**Patent- und Rechtsanwaltskanzlei mbB**
**Berliner Freiheit 2**
**10785 Berlin (DE)**

(54) **POLYSACCHARIDE-CONTAINING PARTICLE PRODUCTION METHOD, PRECURSOR POLYSACCHARIDE-CONTAINING PARTICLES, AND POLYSACCHARIDE-CONTAINING PARTICLES**

(57) According to the present invention, there is provided a method for producing polysaccharide-containing particles comprising a spray drying step and a desalting step, wherein in the spray drying step, a dispersion containing a salt-form polysaccharide is spray dried to obtain precursor polysaccharide-containing particles, and in the desalting step, a desalting agent is brought into contact with the salt-form polysaccharide contained in the precursor polysaccharide-containing particles, and the salt-form polysaccharide is desalted with the desalting agent to obtain polysaccharide-containing particles.

**Description**

TECHNICAL FIELD

The present invention relates to a method for producing polysaccharide-containing particles, precursor polysaccharide-containing particles, and polysaccharide-containing particles.

BACKGROUND ART

Conventionally, particles containing polysaccharides have been used in a wide range of applications as additives for cosmetics, pharmaceuticals, foods, paints, and the like. For example, Patent Literature 1 discloses a method for producing fine particles characterized by granulating an aqueous solution in which a water-soluble polysaccharide and a reactive resin or a water-soluble crosslinking agent are dissolved by spray drying and crosslinking the obtained particles by heat treatment to render them water-insoluble. Patent Literature 2 discloses a method for producing polysaccharide-containing particles comprising: a spray drying step of spray drying a solution containing a polysaccharide and a polyhydric alcohol to obtain a precursor; and a heat treatment step of heating the precursor obtained in the spray drying step to obtain polysaccharide-containing particles.

CITATION LIST

PATENT LITERATURE

Patent Literature 1 JP-A-H8-27277
Patent Literature 2 JP-A-2022-94097

SUMMARY OF INVENTION

TECHNICAL PROBLEM

All of the above-mentioned prior art techniques use a crosslinking agent and insolubilize particles containing a polysaccharide by reacting the polysaccharide with the crosslinking agent, resulting in particles containing the crosslinking agent and a crosslinked structure. In recent years, many industries, including the cosmetics industry, have been required to use materials that are non-toxic to the ecosystem and have high biodegradability, as well as to reduce organic solvent usage. Therefore, techniques for insolubilizing water-soluble polysaccharides by crosslinking, which involve chemical reactions and may impair the degradability of the resulting particles, may be unacceptable. In addition, the insolubilization step may require high-temperature, long-duration heat treatment, which can cause the polysaccharide-containing particles to become colored.

The present invention has been made in view of such circumstances and provides a method for producing polysaccharide-containing particles using a desalting agent.

SOLUTION TO PROBLEM

According to the present invention, there is provided a method for producing polysaccharide-containing particles comprising:

a spray drying step; and
a desalting step,
wherein:

in the spray drying step, a dispersion containing a salt-form polysaccharide is spray dried to obtain precursor polysaccharide-containing particles, and
in the desalting step, a desalting agent is brought into contact with the salt-form polysaccharide contained in the precursor polysaccharide-containing particles, and the salt-form polysaccharide is desalted with the desalting agent to obtain polysaccharide-containing particles.

As a result of intensive studies, the present inventors have found that polysaccharide-containing particles that are poorly soluble in water and have little coloring can be obtained by a method for producing polysaccharide-containing particles comprising a spray drying step of spray drying a dispersion containing a salt-form polysaccharide to obtain precursor polysaccharide-containing particles, and a desalting step of contact a desalting agent with the salt-form polysaccharide and desalting the salt-form polysaccharide with the desalting agent to obtain polysaccharide-containing particles, and have completed the present invention.

Various embodiments of the present invention are exemplified below. The embodiments shown below can be combined with each other.

[1] A method for producing polysaccharide-containing particles comprising: a spray drying step; and a desalting step, wherein:

in the spray drying step, a dispersion containing a salt-form polysaccharide is spray dried to obtain precursor polysaccharide-containing particles, and
in the desalting step, a desalting agent is brought into contact with the salt-form polysaccharide contained in the precursor polysaccharide-containing particles, and the salt-form polysaccharide is desalted with the desalting agent to obtain polysaccharide-containing particles.

[2] The method for producing polysaccharide-containing particles of [1], wherein the desalting step includes a heating step, and, in the heating step, the precursor polysaccharide-containing particles are heated in a state where the desalting agent is in contact with the salt-form polysaccharide, and the salt-form polysaccharide is desalted with the desalting agent to obtain polysaccharide-containing particles.

[3] The method for producing polysaccharide-containing particles of [1], wherein the dispersion further comprises inorganic particles.

[4] The method for producing polysaccharide-containing particles of any one of [1] to [3], wherein the desalting agent is a nitrogen-containing compound, a carboxylic acid, or an alcohol.

[5] The method for producing polysaccharide-containing particles of [4], wherein the nitrogen-containing compound is at least one selected from a group consisting of carbamide, amine, acid amide, and a nitrogen-containing heterocyclic compound.

[6] The method for producing polysaccharide-containing particles of any one of [1] to [5], wherein the polysaccharide is at least one selected from a group consisting of chitosan, cellulose, and derivatives thereof.

[7] The method for producing polysaccharide-containing particles of any one of [1] to [6], wherein a color difference $\Delta E$ of solid content when polysaccharide-containing particles are immersed in water is 0 to 60, and the color difference $\Delta E$ is a value with respect to a standard white in L*a*b* color space.

[8] The method for producing polysaccharide-containing particles of [2], wherein, in the heating step, heating treatment is performed at 100°C or higher and lower than 200°C.

[9] Precursor polysaccharide-containing particles comprising a salt-form polysaccharide and a desalting agent capable of desalting the salt-form polysaccharide.

[10] Polysaccharide-containing particles comprising a polysaccharide and a neutralizing agent, wherein the neutralizing agent is capable of neutralizing an acid or a base capable of forming a salt with the polysaccharide.

[11] The polysaccharide-containing particles of [10], wherein a solubility when immersed in water at 25°C for 7 days is 50% by mass or less.

[12] The polysaccharide-containing particles of [10] or [11], further comprising inorganic particles.

EFFECT OF INVENTION

According to the method for producing polysaccharide-containing particles of the present invention, polysaccharide-containing particles that are poorly soluble in water and have little coloring can be obtained by a mechanism of desalting a salt-form polysaccharide using a desalting agent. Furthermore, the obtained polysaccharide-containing particles can be used in various applications such as external preparations for cosmetics, additives for paints, additives for pharmaceuticals, additives for resin compositions, additives for films for imparting optical or anti-blocking performance, and members for processes such as sintering and polishing, taking advantage of their properties, and can be used particularly for cosmetics.

DESCRIPTION OF EMBODIMENTS

Hereinafter, the present invention will be described in detail by exemplifying embodiments of the present invention. However, the present invention is not limited by these descriptions. The respective features of the embodiments of the present invention shown below can be combined with each other. Furthermore, an invention is established independently for each feature.

1. Method for Producing Polysaccharide-Containing Particles

The method for producing polysaccharide-containing particles according to the present invention comprises a spray drying step and a desalting step and optionally includes a heating step. In the spray drying step, a dispersion containing a salt-form polysaccharide is spray dried to obtain precursor polysaccharide-containing particles. In the desalting step, a desalting agent is brought into contact with the salt-form polysaccharide contained in the precursor polysaccharide-containing particles, and the salt-form polysaccharide is desalted with the desalting agent to obtain polysaccharide-containing particles.

In the production method of the present invention, a desalting agent is brought into contact with a salt-form polysaccharide (a polysaccharide in salt form). In the production method of the present invention, the desalting agent may be added in any step as long as a state where the desalting agent is in contact with the salt-form polysaccharide can be formed in the desalting step.

The desalting agent can be added, for example, in the dispersion preparation step. In this case, in the dispersion preparation step, a dispersion containing a salt-form polysaccharide and a desalting agent is prepared, and the dispersion is spray dried to obtain precursor polysaccharide-containing particles containing the salt-form polysaccharide and the desalting agent. I n this case, the desalting step preferably includes a heating step. When the desalting agent is blended into the dispersion, it is preferable to appropriately select the types of the salt-form polysaccharide and the desalting agent, and as one example, it is preferable to select a combination in which desalting hardly proceeds at room temperature.

The desalting agent can also be added, for example, after obtaining the precursor polysaccharide-containing particles in the spray drying step (in this case, the precursor polysaccharide-containing particles may not contain the desalting agent). In this case, by immersing the obtained precursor polysaccharide-containing particles in a solution containing the desalting agent, spraying and/or applying a solution containing the desalting agent, or exposing them to a gas containing the desalting agent, the desalting agent can be added, and a state where the desalting agent is in contact with the salt-form polysaccharide can be formed.

In one embodiment of the present invention, the desalting agent can be brought into contact after obtaining the precursor polysaccharide-containing particles in the spray drying step. In this case, the heating step can be performed or not performed. In one embodiment of the present invention, water resistance can be improved even when the heating step is not performed thereafter. From the viewpoint of suppressing coloring, it is preferable not to perform the heating step after bringing the desalting agent into contact. When the heating step is not included, it is preferable to appropriately select the types of the salt-form polysaccharide and the desalting agent, and as one example, it is preferable to select a combination in which desalting proceeds at room temperature.

In the production method according to one embodiment of the present invention, it is preferable to include at least one of the above-mentioned desalting agent addition methods. In the production method according to one embodiment of the present invention, it is preferable to bring the desalting agent into contact after obtaining the precursor polysaccharide-containing particles in the spray drying step.

1.1 Dispersion Preparation Step

The method for producing polysaccharide-containing particles according to one embodiment of the present invention may include a dispersion preparation step.

In the dispersion preparation step, a dispersion containing a salt-form polysaccharide is prepared. The dispersion contains a salt-form polysaccharide and may further contain a desalting agent when the desalting agent is added in the dispersion preparation step. The dispersion may also contain inorganic particles.

Examples of the solvent of the dispersion may include water, an organic solvent, and a mixed solvent of water and an organic solvent. Among these, it is preferable to include water, and it is preferable to be water. Examples of water include natural water, purified water, distilled water, ion-exchanged water, pure water, and the like, and among these, ion-exchanged water is preferable. Examples of organic solvents include aliphatic monohydric alcohols such as methanol, ethanol, and isopropyl alcohol, ketones such as acetone, methyl ethyl ketone, and methyl isobutyl ketone, aromatic compounds such as toluene and xylene. The solvent may be, for example, a mixed solvent of water and an organic solvent. The solvent may not contain an alcohol with 3 or more carbon atoms, or may not contain an alcohol at all.

1.1.1 Salt-Form Polysaccharide

The dispersion contains a salt-form polysaccharide. The dispersion may be obtained by adding a salt-form polysaccharide, that is, a polysaccharide salt, to a solvent, or may be obtained by adding a polysaccharide and a salt-forming agent to a solvent. The salt-form polysaccharide may be one kind of compound. The salt-form polysaccharide may contain two or more kinds of compounds.

Examples of the polysaccharide include glucosamine-based polysaccharides and molecules containing a glucose unit. Examples of glucosamine-based polysaccharides include chitosan, which is β-1,4-glucosamine. In addition, they include chitin, which is β-1,4-N-acetylglucosamine. Examples of molecules containing a glucose unit include β-glucan. Examples of β-glucan include β-1,4-glucan and β-1,3-glucan. Examples of β-1,4-glucan include cellulose, cellulose acetate, ethyl cellulose, methyl cellulose, carboxymethyl cellulose, carboxyethyl cellulose, hydroxyethyl cellulose, and hydroxypropyl methyl cellulose. Examples of β-1,3-glucan include curdlan and paramylon. Also, alginic acid, which is a block polymer of β-D-mannuronic acid and α-L-guluronic acid, can be exemplified. Furthermore, derivatives of these can be mentioned. It is preferable that the polysaccharide is at least one selected from a group consisting of chitosan, cellulose, and derivatives thereof.

Examples of the polysaccharide salt include the salts of the polysaccharides described above, and examples of the salt-forming agent include an acid or a base capable of forming the salt state with the polysaccharide to be used. As one example, when the polysaccharide is chitosan, an acid can be used as the salt-forming agent. Examples of the acid include organic acids such as carboxylic acids, and inorganic acids such as hydrochloric acid and sulfuric acid. Examples of the carboxylic acids include the carboxylic acids listed as the desalting agent described later. As another example, when the polysaccharide is carboxymethyl cellulose, a base can be used as the salt-forming agent. Examples of the base include nitrogen-containing compounds, and inorganic bases such as aluminum hydroxide and potassium hydroxide. Examples of the nitrogen-containing compound include the nitrogen-containing compounds listed as the desalting agent described later.

The salt-form polysaccharide is preferably water-soluble. The salt-form polysaccharide can have higher solubility than the desalted polysaccharide contained in polysaccharide-containing particles described later. Specifically, the salt-form polysaccharide can have a solubility of 80 % by mass or more when immersed in water at 25°C for 7 days. The solubility of the salt-form polysaccharide when immersed in water at 25°C for 7 days is, for example, 80, 85, 90, 95, 96, 97, 98, 99, or 100 % by mass, and may be in the range between the two values exemplified herein.

1.1.2 Desalting Agent

When the desalting agent is added in a dispersion preparation step, the dispersion can contain the desalting agent, and the desalting agent is not particularly limited as long as it is a compound that neutralizes and desalts an acid or base that is a salt-forming agent that forms a salt with the polysaccharide, or a compound that promotes desalting. The desalting agent may include those that do not have a desalting function under normal temperature and atmospheric pressure environment but exhibit a desalting function by applying energy such as heating or light. The desalting agent can be a base, an acid, or an alcohol, and can be a base, an acid, or an alcohol having 3 or more carbon atoms. Examples of the desalting agent that exhibits a desalting function by applying energy such as heating or light include a photo (thermal) base generator and a photo (thermal) acid generator, and an example thereof is an epoxy resin curing agent. The desalting agent can be a base when the salt-forming agent is an acid, and the desalting agent can be an acid when the salt-forming agent is a base. Although the mechanism by which alcohol functions as a desalting agent is not necessarily clear, it is presumed that one of the factors is that crystal structure changes by an addition of alcohol. The desalting agent can be a nitrogen-containing compound, a carboxylic acid, or an alcohol. The desalting agent can be a nitrogen-containing compound, a carboxylic acid, or a monovalent alcohol. The desalting agent can be a nitrogen-containing compound, a carboxylic acid, or a monovalent alcohol having 3 or more carbon atoms. When performing a heating step described later, it is more preferable that the desalting agent has heat resistance that remains in the obtained polysaccharide-containing particles.

The nitrogen-containing compound can be at least one selected from a group consisting of carbamide, amine, acid amide, and a nitrogen-containing heterocyclic compound. Examples of the carbamide include urea and urea derivatives. Specifically, examples thereof include urea, methylurea, ethylurea, propylurea, butylurea, isobutylurea, 1,1-dimethylurea, 1,3-dimethylurea, 1,1-diethylurea, 1,3-diethylurea, tetramethylurea, 1,1,3,3-tetraethylurea, and 1,1,3,3-tetrabutylurea. Among them, urea and tetramethylurea are particularly preferable from the viewpoint of a desalting effect.

Examples of the amine include compounds having an amine structure, and examples thereof include alkylamines such as ammonia; methylamine, ethylamine, n-propylamine, n-butylamine, n-amylamine, and n-hexylamine; alkanolamines such as methanolamine, triethanolamine, diethanolamine, monoethanolamine, propanolamine, isopropanolamine, and diisopropanolamine; and polyamines such as ethylenediamine, putrescine, cadaverine, hexamethylenediamine, and polyethylene diamine. Examples of the acid amide include compounds having a structure in which ammonia or a primary or secondary amine is dehydrated and condensed with an oxo acid, and examples include niacinamide, acetanilide, acetamide, ε-caprolactam, and γ-butyrolactam.

Examples of the nitrogen-containing heterocyclic compound include pyridines such as pyridine, niacin, 2-methylpyridine, 3-methylpyridine, 4-methylpyridine, 2-hydroxy-4-methylpyridine, 2-hydroxy-6-methylpyridine, 2-hydroxypyridine, 3-hydroxypyridine, and 4-hydroxypyridine; pyrrolidines such as 1-methylpyrrolidine, 1-ethylpyrrolidine, 1-(2-hydroxyethyl)pyrrolidine, and 2-(2-hydroxyethyl)-1-methylpyrrolidine; piperidines such as 1-methylpiperidine, 1-ethylpiperidine, 1-(2-hydroxyethyl)piperidine, 1-(hydroxymethyl)piperidine, 3-hydroxy-1-methylpiperidine, 4-hydroxy-1-methylpiperidine, and 1,4-dimethylpiperidine; piperazines such as 1-methylpiperazine and 1-ethylpiperazine; natural extracts containing a nitrogen-containing heterocycle; and nucleic acids such as purine bases and pyrimidine bases; and niacin is particularly preferable from the viewpoint of a desalting effect.

The nitrogen-containing compound preferably contains one or more selected from polyethylene diamine, ammonia, urea, tetramethylurea, and niacin from the viewpoint of a desalting effect. The desalting agent, particularly the nitrogen-containing compound, preferably has a pH of 7 or more. The pH is, for example, 7, 8, 9, 10, 11, 12, 13, or 14, and may be in the range between the two values exemplified herein. The nitrogen-containing compound is preferably strongly basic, and polyethylene diamine and ammonia are more preferable.

Examples of the carboxylic acid include saturated fatty acids such as formic acid, acetic acid, propionic acid, and butyric acid, hydroxy acids such as lactic acid, malic acid, and citric acid, and dicarboxylic acids such as oxalic acid, succinic acid, and adipic acid.

Examples of the alcohol include monovalent alcohols such as 1-propyl alcohol, isopropyl alcohol, and 1-butanol; dihydric alcohols such as ethylene glycol, 1,2-propanediol, 1,3-propanediol, 1,4-butanediol, neopentyl glycol, 1,6-hexanediol, diethylene glycol, triethylene glycol, tetraethylene glycol, dipropylene glycol, polyethylene glycol, and polypropylene glycol; and trihydric or higher polyhydric alcohols such as glycerin, diglycerin, erythritol, trimethylolethane, trimethylolpropane, pentaerythritol, dipentaerythritol, and sorbitol. From the viewpoint of a desalting effect, a monovalent alcohol is preferable, and isopropyl alcohol is more preferable. Methanol and ethanol are not preferable because of their poor desalting effect. Since alcohol may volatilize due to heat during spray-drying and a sufficient desalting effect may not be obtained, it is preferable to bring the desalting agent into contact with the precursor polysaccharide-containing particles after obtaining the precursor polysaccharide-containing particles in a spray drying step.

With a desalting agent other than carbamide, water resistance can be improved by obtaining the precursor polysaccharide-containing particles in the spray drying step, and then bringing the desalting agent into contact with the precursor polysaccharide-containing particles and the subsequent heating step can be optional. When carbamide is used as the desalting agent, it is preferable to perform the heating step.

When performing spray-drying by putting the desalting agent in the dispersion, the desalting agent preferably has a boiling point of 70°C or more. In this case, the boiling point of the desalting agent is, for example, 70, 80, 90, 100, 110, 120, 130, 140, 150, 160, 170, 180, 190, or 200°C, and may be in the range between the two values exemplified herein.

When bringing the desalting agent into contact with the precursor polysaccharide-containing particles after obtaining the precursor polysaccharide-containing particles in the spray drying step, a desalting agent with low water solubility can also be used. When bringing the desalting agent into contact with the precursor polysaccharide-containing particles after obtaining the precursor polysaccharide-containing particles in the spray drying step, the desalting agent is more preferably polyethylene diamine, ammonia, isopropyl alcohol, an aqueous alkali metal salt solution, an aqueous alkaline earth metal solution, a tertiary amine such as tetrahydroxypropylethylenediamine or dimethylaminoethanol, or a primary amine such as 1-amino-3-undecanoxy-propane.

1.1.3 Inorganic Particles

The dispersion may contain inorganic particles. Examples of the inorganic particles include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, biotite, lepidolite, silicic acid, silicic anhydride, aluminum silicate, magnesium silicate, aluminum magnesium silicate, calcium silicate, barium silicate, strontium silicate, metal tungstate, hydroxyapatite, vermiculite, Higilite, bentonite, montmorillonite, hectorite, zeolite, dicalcium phosphate, alumina, aluminum hydroxide, boron nitride, boron nitride, and silica.

The inorganic particles are preferably plate-shaped or scale-shaped, and more preferably mica. The mica may be natural mica or synthetic mica.

The average particle diameter of the inorganic particles can be 5 to 100 $\mu$m, for example, 5, 10, 15, 20, 25, 30, 35, 40, 45, 50, 55, 60, 65, 70, 75, 80, 85, 90, 95, or 100 $\mu$m, and may be within the range between any two of the numerical values exemplified here. The average particle diameter can be a volume average diameter by a laser diffraction scattering method. The aspect ratio of the inorganic particles can be 20 to 200, for example, 20, 40, 60, 80, 100, 120, 140, 160, 180, or 200, and may be within the range between any two of the numerical values exemplified here. The aspect ratio can be defined as the ratio of the major axis to the thickness of the inorganic particles.

1.1.4 Content of Each Component in Dispersion

The dispersion can contain 0.05 to 35% by mass of the salt-form polysaccharide. The concentration of the polysaccharide salt in the dispersion is, for example, 0.05, 0.1, 0.5, 1, 5, 10, 15, 20, 25, 30, or 35 % by mass, and may be within the range between any two of the numerical values exemplified here. When obtaining the dispersion by adding the polysaccharide and the salt-forming agent to a solvent, 1 to 300 parts by mass of the salt-forming agent can be added with respect to 100 parts by mass of the polysaccharide. The content of the salt-forming agent with respect to 100 parts by mass of the polysaccharide is, for example, 0, 50, 100, 150, 200, 250, or 300 parts by mass, and may be within the range between any two of the numerical values exemplified here.

When the dispersion contains the desalting agent, the dispersion can contain 0.1 to 10.0% by mass of the desalting agent. The concentration of the desalting agent in the dispersion is, for example, 0.1, 0.2, 0.3, 0.5, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, or 10.0 % by mass, and may be within the range between any two of the numerical values exemplified here. The dispersion preferably contains 1 part by mass or more of the desalting agent with respect to 100 parts by mass of the salt-form polysaccharide. The content of the desalting agent with respect to 100 parts by mass of the salt-form polysaccharide in the dispersion is, for example, 1, 2, 3, 5, 10, 15, 20, 25, or 30 parts by mass, and may be within the range between any two of the numerical values exemplified here. The desalting agent plays a role in promoting the desalting of the salt-form polysaccharide, and from the viewpoint that it preferably remains as a neutralizing agent in the polysaccharide-containing particles thereafter, it is preferable to add it in excess so that it remains through the production step according to one embodiment of the present invention, and so that it remains through the heating step when performing the heating step.

When the dispersion contains inorganic particles (for example, mica), the concentration of the inorganic particles in the dispersion can be 0.1 to 70% by mass. The concentration of the inorganic particles is, for example, 0.1, 0.5, 1, 5, 10, 15, 20, 25, 30, 35, 40, 45, 50, 60, or 70% by mass, and may be in the range between the two values exemplified herein. The dispersion preferably contains 10 parts by mass or more of the salt-form polysaccharide with respect to 100 parts by mass of the inorganic particles. The content of the salt-form polysaccharide with respect to 100 parts by mass of the inorganic particles in the dispersion is, for example, 10, 15, 20, 25, 30, 35, 40, 45, 50, 55, 60, 65, 70, 75, 80, 85, 90, 95, 100, 110, 120, 130, 140, 150, 200, 250, 300, 350, 400, 450, 500, 550, 600, 650, 700, 750, 800, 850, 900, 950, 1000, 1050, 1100, 1150, or 1200 parts by mass, and may be within the range between any two of the numerical values exemplified here. By adjusting the type and amount of the inorganic particles and the salt-form polysaccharide in the dispersion, the structure and physical properties of the obtained polysaccharide-containing particles can be adjusted. For example, by sufficiently increasing the concentration of the salt-form polysaccharide in the dispersion and sufficiently increasing the content of the salt-form polysaccharide with respect to the inorganic particles, it is possible to obtain polysaccharide-containing particles containing inorganic particles sufficiently coated with a coating layer containing a polysaccharide. As one example, particularly when the inorganic particles are plate-shaped particles or scale-shaped particles such as mica, it is possible to obtain polysaccharide-containing particles that are composite particles in which a sufficient number and size of polysaccharide-containing spherical particles are attached to the surface of the inorganic particles or the coating layer. Inorganic particles sufficiently coated with a coating layer, and composite particles in which a sufficient number and size of polysaccharide-containing spherical particles are attached to the surface of the inorganic particles or the coating layer, can form a powder having a low average coefficient of friction and a high soft focus factor.

The dispersion may contain known components within a range that does not impair the effects of the present invention. Examples of the known components include components that the precursor polysaccharide-containing particles described later may contain.

The viscosity of the dispersion is preferably 1 Pa·s or less. By setting the viscosity to such a value, it is possible to obtain polysaccharide-containing particles having an appropriate shape and/or structure. In the dispersion preparation step, a process of adjusting the viscosity and rheological properties of the dispersion may be performed as necessary. For example, examples thereof include a process for lowering the viscosity, particularly the dynamic viscosity at high shear, and a process for imparting pseudoplasticity or thixotropy, and as one example, these processes can be performed by adding a dispersant or reducing the molecular weight of an organic material.

The dispersion can be prepared by stirring with a known stirrer, and for example, a disper mixer, a homomixer, a high-pressure homogenizer, or the like can be used.

The order of mixing in the dispersion preparation step is not particularly limited. As one example, when the dispersion contains the salt-form polysaccharide and the desalting agent, the polysaccharide salt (or the polysaccharide and the salt-forming agent) is added to a solvent and the mixture is mixed, and after preparing a dispersion containing the salt-form polysaccharide first, the desalting agent may be added. By performing mixing in such an order, precipitation of the polysaccharide can be prevented.

1.2 Spray Drying Step

In the spray drying step, a dispersion containing a salt-form polysaccharide is spray dried to obtain precursor polysaccharide-containing particles. The precursor polysaccharide-containing particles according to the present invention contain the salt-form polysaccharide, may contain the desalting agent capable of desalting the salt-form polysaccharide, and may further contain the inorganic particles.

In the spray drying step, the dispersion is supplied to a spray dryer adjusted to a predetermined temperature and sprayed to obtain the precursor polysaccharide-containing particles. The spray dryer is not particularly limited as long as it is a spray dryer used in normal granulation production. The spray dryer may include:

- a raw material tank for storing the dispersion;
- a raw material supply pump for supplying the dispersion;
- a nozzle (four-fluid nozzle, two-fluid nozzle, or one-fluid nozzle) or a rotating disk for turning the dispersion into fine droplets;
- a drying chamber for drying and granulating the droplets;
- a blower, filter, and heater for blowing dried and heated air or inert gas into the drying chamber;
- a collector for collecting the composite powder precursor by a two-point collection method, a cyclone method, a bag filter method, or the like (these may be collectors that can be heated).

The shape, structure and physical properties of the obtained precursor polysaccharide-containing particles can be adjusted by adjusting the droplet formation conditions and drying conditions in the spray drying step.

In the spray drying step, the outlet temperature of the spray dryer can be 70 to 250°C The outlet temperature is, for example, 70, 80, 90, 100, 110, 120, 130, 140, 150, 160, 170, 180, 190, 200, 210, 220, 230, 240, or 250°C, and may be within the range between any two of the numerical values exemplified here. When the dispersion contains the desalting agent, in the spray drying step, a part of the salt-form polysaccharide in the obtained precursor polysaccharide-containing particles may be desalted by the desalting agent.

That is, the spray drying step can also serve as a desalting step described later. The spray drying step can also serve as a heating step described later, for example, by adjusting the drying conditions after granulation, or by providing the spray dryer with a heating function or a heating chamber capable of heating and holding for a certain period of time.

3.3 Desalting Step

In the desalting step, the salt-form polysaccharide contained in the precursor polysaccharide-containing particles is brought into contact with the desalting agent, and the salt-form polysaccharide is desalted with the desalting agent to obtain polysaccharide-containing particles.

The desalting step may involve heating and may include a heating step described below.

In general, methods for improving water resistance by forming a crosslinked structure using a crosslinking agent, or methods for improving water resistance by modifying the entire material or the surface of the material are known. However, these methods may not be acceptable because they involve chemical reactions and may deteriorate the degradability of the obtained particles. In the present invention, in the heating step, the salt-form polysaccharide is brought into contact with a desalting agent, the salt-form polysaccharide is desalted with the desalting agent, and the crystallinity of the polysaccharide is increased, thereby improving water resistance (lowering solubility). According to the present invention, it is presumed that by contacting a desalting agent, desalting and/or crystallinity improvement of the salt-form polysaccharide is promoted, and a strong and robust state in which polysaccharide molecules are more densely hydrogen-bonded to each other is easily created, and water resistance can be improved. According to one embodiment of the present invention, yellowing of the material due to heating can be prevented. In addition, compared to the case where a desalting agent is not added, water resistance can be improved without heating treatment or by heating treatment at a low temperature and/or for a short time, so that yellowing of the material can be prevented.

As described above, in the production method of the present invention, the desalting agent may be added in any step as long as a state in which the salt-form polysaccharide is in contact with the desalting agent can be formed. The desalting agent may be added after obtaining the precursor polysaccharide-containing particles in the spray drying step. The production method according to one embodiment of the present invention may include a desalting agent addition step before and/or during the heating step. In this case, in the desalting agent addition step, for example, by immersing the obtained precursor polysaccharide-containing particles in a solution containing the desalting agent, or by spraying and/or applying a solution containing the desalting agent, or by exposing the obtained precursor polysaccharide-containing particles to a gas containing the desalting agent, the desalting agent is added, and a state in which the salt-form polysaccharide is brought into contact with the desalting agent can be formed. Examples of the desalting agent include the desalting agents listed in the dispersion preparation step. When preparing a solution containing the desalting agent, the solution can contain 0.1 to 90.0% by mass of the desalting agent. The concentration of the desalting agent in the solution is, for example, 0.1, 0.2, 0.3, 0.5, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 20.0, 30.0, 40.0, 50.0, 60.0, 70.0, 80.0, or 90.0% by mass, and may be within the range between any two of the numerical values exemplified here. The amount of the desalting agent to be added is preferably 10 parts by mass or more, more preferably 40 parts by mass or more, and most preferably 100 parts by mass or more, with respect to 100 parts by mass of the salt-form polysaccharide contained in the precursor polysaccharide-containing particles. The upper limit is preferably 400 parts by mass or less, more preferably less than 200 parts by mass, and most preferably 180 parts by mass or less.

The content of the desalting agent with respect to 100 parts by mass of the salt-form polysaccharide contained in the precursor polysaccharide-containing particles is, for example, 1, 2, 3, 5, 10, 15, 20, 25, 30, 50, 100, 200, 300, or 400 parts by mass, and may be within the range between any two of the numerical values exemplified here. The upper limit is preferably 400 parts by mass or less, more preferably less than 200 parts by mass, and most preferably 180 parts by mass or less. When the desalting agent contains a base (or an acid), the amount of the base (or acid) in the solution containing the desalting agent, which is brought into contact with the precursor polysaccharide-containing particles, with respect to 100 parts by mass of the salt-form polysaccharide contained in the precursor polysaccharide-containing particles can be 1 to 150 parts by mass, for example, 1, 2, 3, 5, 10, 15, 20, 25, 30, 40, 50, 60, 70, 80, 90, 100, 110, 120, 130, 140, or 150 parts by mass, and may be within the range between any two of the numerical values exemplified here.

As described above, the desalting step can include a heating step, and in the heating step, the precursor polysaccharide-containing particles are heated in the state where the salt-form polysaccharide is in contact with the desalting agent, and the salt-form polysaccharide is desalted with the desalting agent to obtain polysaccharide-containing particles.

In the heating step, the heating treatment is preferably performed at 100°C or more and less than 200°C.

The heating temperature is, for example, 100, 105, 110, 115, 120, 125, 130, 135, 140, 145, 150, 155, 160, 165, 170, 175, 180, 185, 190, 195°C, or less than 200°C, and may be within the range between any two of the numerical values exemplified here. The heating time is, for example, 1, 5, 10, 20, 30, 40, 50, 60, 70, 80, 90, 100, 110, 120, 130, 140, 150, 160, 170, 180, 190, 200, 210, 220, 230, 240, 250, 260, 270, 280, 290, or 300 minutes, and may be within the range between any two of the numerical values exemplified here.

As one example, the heating treatment can be performed under the following condition A.

A. 100°C or more and less than 150°C for 20 minutes or more

The heating temperature of condition A can be 100°C or more and less than 150°C, for example, 100, 105, 110, 115, 120, 125, 130, 135, 140, 145°C, or less than 150°C, and may be within the range between any two of the numerical values exemplified here. The heating time of condition A can be 20 minutes or more. The heating time may be, for example, 20, 30, 40, 50, 60, 70, 80, 90, 100, 110, 120, 130, 140, 150, 160, 170, 180, 190, 200, 210, 220, 230, 240, 250, 260, 270, 280, 290, or 300 minutes, and may be within the range between any two of the numerical values exemplified here.

As another example, the heating treatment can be performed under the following condition B.

B. 150°C or more and less than 200°C for 1 minute or more and less than 90 minutes

The heating temperature of condition B can be 150°C or more and less than 200°C, for example, 150, 155, 160, 165, 170, 175, 180, 185, 190, 195°C, or less than 200°C, and may be within the range between any two of the numerical values exemplified here. The heating time of condition B can be 1 minute or more and less than 90 minutes. The heating time may be, for example, 1, 2, 3, 5, 10, 15, 20, 25, 30, 35, 40, 45, 50, 55, 60, 65, 70, 75, 80, 85 minutes, or less than 90 minutes, and may be within the range between any two of the numerical values exemplified here.

Yellowing and coloring of the precursor polysaccharide-containing particles can be prevented by setting the conditions to a low temperature for a long time or a high temperature for a short time. The heating treatment conditions are preferably set to a temperature and time such that the color difference $\Delta E$ of the solid content when the obtained polysaccharide-containing particles are immersed in water is 0 to 60. The color difference $\Delta E$ of the solid content when the obtained polysaccharide-containing particles are immersed in water is, for example, 0, 5, 10, 15, 20, 25, 30, 35, 40, 45, 50, 55, or 60, and the heating treatment conditions are preferably set to a temperature and time such that the color difference $\Delta E$ of the solid content is within the range between the two values exemplified herein.

The production method according to one embodiment of the present invention can include a washing step after the desalting step. In the washing step, excess desalting agent can be removed by dehydration washing. The dehydration step may not be performed, and the desalting agent may remain after the dehydration step.

Hereinafter, the precursor polysaccharide-containing particles and the polysaccharide-containing particles according to the present invention will be described.

The precursor polysaccharide-containing particles can be obtained by spray drying the dispersion containing the salt-form polysaccharide (and the desalting agent as needed) in the spray drying step described above.

2. Precursor Polysaccharide-containing Particles

The precursor polysaccharide-containing particles according to the present invention contain a salt-form polysaccharide. The precursor polysaccharide-containing particles according to one embodiment of the present invention contain a salt-form polysaccharide and a desalting agent capable of desalting the salt-form polysaccharide. When the precursor polysaccharide-containing particles according to one embodiment of the present invention contain a desalting agent, a part of the salt-form polysaccharide in the precursor polysaccharide-containing particles may be desalted by the desalting agent, that is, may contain a desalted polysaccharide. Examples of the salt-form polysaccharide, the desalting agent, and the polysaccharide include the salt-form polysaccharide, the desalting agent, and the polysaccharide listed in the description of the dispersion preparation step of the method for producing polysaccharide-containing particles.

When the precursor polysaccharide-containing particles contain the desalting agent, the precursor polysaccharide-containing particles preferably contain 0.1 parts by mass or more of the desalting agent with respect to 100 parts by mass of the total of the salt-form polysaccharide and the polysaccharide in the precursor polysaccharide-containing particles. The content of the desalting agent with respect to 100 parts by mass of the salt-form polysaccharide in the dispersion is, for example, 0.1, 0.2, 0.3, 0.5, 1, 2, 3, 5, 10, 15, 20, 25, or 30 parts by mass, and may be within the range between any two of the numerical values exemplified here.

The precursor polysaccharide-containing particles can be spherical particles. The spherical particles also include substantially spherical particles and rounded particles. The ratio of the maximum diameter of the spherical particle to the minimum diameter of the spherical particle is preferably 0.5 to 1.5, and more preferably 0.8 to 1.2.

The spherical particles may include spherical particles having a partially crushed or cut spherical shape, such as hemispherical particles (lens-like particles, and the like), but at least half of the spherical particles preferably have the ratio of the maximum diameter of the spherical particle to the minimum diameter of the spherical particle in the above numerical range. The spherical particles may have few dents or wrinkles on the surface and the surface is smooth.

The average particle diameter of the spherical particles can be 10 μm or less. The average particle diameter can be 0.1 to 10 μm, for example, 0.1, 0.5, 1, 2, 3, 4, 5, 6, 7, 8, 9, or 10 μm, and may be within the range between any two of the numerical values exemplified here. The average particle diameter of the spherical particles can be measured by SEM observation. The average particle diameter can be obtained by measuring the particle diameter of each spherical particle and taking the average value thereof.

The precursor polysaccharide-containing particles may further contain inorganic particles. Examples of the inorganic particles include the inorganic particles listed in the description of the dispersion preparation step of the method for producing polysaccharide-containing particles.

When the precursor polysaccharide-containing particles contain inorganic particles (for example, mica), the precursor polysaccharide-containing particles preferably contain 10 parts by mass or more of the total of the salt-form polysaccharide and the polysaccharide with respect to 100 parts by mass of the inorganic particles. The total content of the salt-form polysaccharide and the polysaccharide with respect to 100 parts by mass of the inorganic particles in the dispersion is, for example, 10, 15, 20, 25, 30, 35, 40, 45, 50, 55, 60, 65, 70, 75, 80, 85, 90, 95, 100, 110, 120, 130, 140, 150, 200, 250, 300, 350, 400, 450, 500, 550, 600, 650, 700, 750, 800, 850, 900, 950, 1000, 1050, 1100, 1150, or 1200 parts by mass, and may be within the range between any two of the numerical values exemplified here.

When the precursor polysaccharide-containing particles contain inorganic particles, the precursor polysaccharide-containing particle can be composite particle containing an inorganic particle, a coating layer, and a spherical particle. Here, the coating layer can coat the inorganic particle. The spherical particle may be attached to the inorganic particle or the coating layer coating the inorganic particle. The coating layer and the spherical particle may contain the salt-form polysaccharide, may contain the desalting agent capable of desalting the salt-form polysaccharide, and may contain the desalted polysaccharide.

By adjusting the type and amount of the inorganic particles, and the salt-form polysaccharide and the polysaccharide in the precursor polysaccharide-containing particles, the structure and physical properties of the obtained polysaccharide-containing particles can be adjusted. For example, by sufficiently increasing the concentration of the salt-form polysaccharide and the polysaccharide in the precursor polysaccharide-containing particles, and sufficiently increasing the content of the salt-form polysaccharide and the polysaccharide with respect to the inorganic particles, it is possible to obtain precursor polysaccharide-containing particles containing inorganic particles sufficiently coated with a coating layer containing the salt-form polysaccharide and the polysaccharide. According to the precursor polysaccharide-containing particles, it is possible to obtain polysaccharide-containing particles that can form a powder having a high soft focus factor. As one example, particularly when the inorganic particles are plate-shaped particles or scale-shaped particles such as mica, it is possible to obtain precursor polysaccharide-containing particles that are composite particles in which a sufficient number and size of precursor polysaccharide-containing spherical particles are attached to the surface of the inorganic particles or the coating layer. According to the precursor polysaccharide-containing particles, it is possible to obtain polysaccharide-containing particles that can form a powder having a low average coefficient of friction and a high soft focus factor.

When the precursor polysaccharide-containing particles contain inorganic particles, it is preferable that at least a part of the inorganic particle is coated with the coating layer, and that the whole is coated with the coating layer. As described above, the coating layer contains the salt-form polysaccharide, may contain the desalting agent capable of desalting the salt-form polysaccharide, and may contain the desalted polysaccharide. Whether the inorganic particles are coated with the coating layer can be confirmed by SEM observation, EDS (energy dispersive X-ray analysis), re-dissolving the coating layer coating the inorganic particles and confirming its mass, and the like. According to the precursor polysaccharide-containing particles of the present invention, it is possible to obtain polysaccharide-containing particles containing the inorganic particles coated with the coating layer containing the polysaccharide through a heating step. Such polysaccharide-containing particles can form a powder having excellent soft-focus properties because the inorganic particles are coated with the coating layer.

When the precursor polysaccharide-containing particles contain inorganic particles, at least one spherical particle can be attached to the inorganic particle and/or the coating layer, and for example, when the inorganic particle is plate-shaped or scale-shaped particle such as mica, it is preferable that at least one spherical particle is attached to both main surfaces of the inorganic particle, directly or via the coating layer. Hereinafter, the particle attached to the inorganic particle means the particle attached to the inorganic particle directly or via the coating layer. The number of spherical particles attached to one inorganic particle is, for example, 1, 2, 3, 5, 9, 10, 15, 20, 30, 31, 40, 50, 60, 70, 80, 90, or 100, and may be within the range between any two of the numerical values exemplified here. The presence or absence of spherical particle attached to the inorganic particle, and the number of spherical particles attached to one inorganic particle can be confirmed by observing the polysaccharide-containing particles with SEM. A part of the spherical particles may not be attached to the inorganic particles, and the precursor polysaccharide-containing particles containing inorganic particles according to one embodiment of the present invention can also contain the spherical particle that are not attached to the inorganic particles.

The precursor polysaccharide-containing particles according to one embodiment of the present invention can contain 50 parts by mass or more of the total of the salt-form polysaccharide, the polysaccharide, the salt-forming agent (and inorganic particles as needed) with respect to 100 parts by mass of the precursor polysaccharide-containing particles, the content may be for example, 50, 60, 70, 80, 90, or 100 parts by mass, and may be within the range between any two of the numerical values exemplified here. The precursor polysaccharide-containing particles according to one embodiment of the present invention can also be composed of the salt-form polysaccharide, the polysaccharide, the salt-forming agent (and inorganic particles as needed).

The precursor polysaccharide-containing particles according to one embodiment of the present invention may contain known components used in polysaccharide-containing particles, for example, known components used in cosmetics, within a range that does not impair the effects of the present invention. Examples of known components include organic powders, oily components, surfactants, ultraviolet absorbers, humectants, anti-fading agents, antioxidants, antifoaming agents, preservatives, fragrances, solubilizers, plasticizers, viscosity modifiers, skin beautifying ingredients (whitening agents, cell activators, skin roughness improvers, blood circulation promoters, skin astringents, anti-seborrheic agents, and the like), vitamins, amino acids, antiperspirants, alcohols, film formers, antiinflammatory agents, cooling agents, nucleic acids, hormones, clathrate compounds, pH adjusters, chelating agents, and the like.

3. Polysaccharide-Containing Particles

Polysaccharide-containing particles can be obtained by bringing a desalting agent into contact with a salt-form polysaccharide and desalting the salt-form polysaccharide with the desalting agent. The polysaccharide-containing particles according to the present invention contain a polysaccharide and a neutralizing agent. Here, the neutralizing agent is capable of neutralizing an acid or a base capable of forming a salt with a polysaccharide. The neutralizing agent can be the desalting agent that is contained in the precursor polysaccharide-containing particles and remains in the polysaccharide-containing particles even after the desalting step or the heating step. The polysaccharide-containing particles may contain the salt-form polysaccharide that has not been desalted in the desalting step or the heating step, but from the viewpoint of improving water resistance, it is preferable that the content of the salt-form polysaccharide is small. The degree of the content of the salt-form polysaccharide can be confirmed by the evaluation of solubility described later. The polysaccharide-containing particles may not contain the salt-form polysaccharide. Examples of the polysaccharide and the salt-form polysaccharide include the polysaccharide and the salt-form polysaccharide listed in the description of the dispersion preparation step of the method for producing polysaccharide-containing particles. Examples of the neutralizing agent include the desalting agents listed in the description of the dispersion preparation step of the method for producing polysaccharide-containing particles.

The desalted polysaccharide preferably has a solubility of less than 80% by mass when immersed in water at 25°C for 7 days. The solubility of the desalted polysaccharide when immersed in water at 25°C for 7 days is, for example, 0, 5, 10, 15, 20, 25, 30, 35, 40, 45, 50, 55, 60, 65, 70, or 75% by mass, and less than 80% by mass, and may be in the range between the two values exemplified herein.

The polysaccharide-containing particles preferably contain 0.01 parts by mass or more of the desalting agent with respect to 100 parts by mass of the total of the polysaccharide and the salt-form polysaccharide in the polysaccharide-containing particles. The content of the desalting agent with respect to 100 parts by mass of the salt-form polysaccharide in the dispersion is, for example, 0.01, 0.02, 0.03, 0.05, 0.1, 0.2, 0.3, 0.5, 1, 2, 3, 5, 10, 15, 20, 25, or 30 parts by mass, and may be within the range between any two of the numerical values exemplified here.

The polysaccharide-containing particles can be obtained by desalting the salt-form polysaccharide with the desalting agent, and the shape and average particle diameter can be the same as the shape and average particle diameter of the precursor polysaccharide-containing particles.

The polysaccharide-containing particles may further contain inorganic particles. Examples of the inorganic particles include the inorganic particles listed in the description of the dispersion preparation step of the method for producing polysaccharide-containing particles.

When the polysaccharide-containing particles contain inorganic particles (for example, mica), the polysaccharide-containing particles preferably contain 10 parts by mass or more of the total of the polysaccharide and the salt-form polysaccharide with respect to 100 parts by mass of the inorganic particles. The total content of the polysaccharide and the salt-form polysaccharide with respect to 100 parts by mass of the inorganic particles in the dispersion is, for example, 10, 15, 20, 25, 30, 35, 40, 45, 50, 55, 60, 65, 70, 75, 80, 85, 90, 95, 100, 110, 120, 130, 140, 150, 200, 250, 300, 350, 400, 450, 500, 550, 600, 650, 700, 750, 800, 850, 900, 950, 1000, 1050, 1100, 1150, or 1200 parts by mass, and may be within the range between any two of the numerical values exemplified here.

When the polysaccharide-containing particles contain inorganic particles, the polysaccharide-containing particle can be composite particle containing an inorganic particle, a coating layer, and a spherical particle. Here, the coating layer can coat the inorganic particle. The spherical particle may be attached to the inorganic particle or the coating layer coating the inorganic particle. The coating layer and the spherical particle contain the polysaccharide and the neutralizing agent capable of neutralizing an acid or a base capable of forming a salt with the polysaccharide and may contain a salt-form polysaccharide.

By adjusting the type and amount of the inorganic particles, and the polysaccharide and the salt-form polysaccharide in the polysaccharide-containing particles, the structure and physical properties of the polysaccharide-containing particles can be adjusted. For example, by sufficiently increasing the concentration of the polysaccharide and the salt-form polysaccharide in the polysaccharide-containing particles and sufficiently increasing the content of the polysaccharide and the salt-form polysaccharide with respect to the inorganic particles, it is possible to obtain polysaccharide-containing particles containing inorganic particles sufficiently coated with a coating layer containing the polysaccharide and the salt-form polysaccharide. According to the polysaccharide-containing particles, it is possible to form a powder having a high soft focus factor. As one example, particularly when the inorganic particles are plate-shaped particles or scale-shaped particles such as mica, the polysaccharide-containing particles are composite particles in which a sufficient number and size of polysaccharide-containing spherical particles are attached to the surface of the inorganic particles or the coating layer. According to the polysaccharide-containing particles, it is possible to form a powder having a low average coefficient of friction and a high soft focus factor.

When the polysaccharide-containing particles contain inorganic particles, it is preferable that at least a part of the inorganic particle is coated with the coating layer, and that the whole is coated with the coating layer. As described above, the coating layer contains a polysaccharide and a neutralizing agent capable of neutralizing an acid or a base capable of forming the salt with the polysaccharide and may contain a salt-form polysaccharide. Whether the inorganic particles are coated with the coating layer can be confirmed by SEM observation, EDS (energy dispersive X-ray analysis), separating the precursor polysaccharide-containing particles and re-dissolving the coating layer coating the inorganic particles to confirm its mass, and the like. Since the inorganic particles are coated with the coating layer, the polysaccharide-containing particles according to the present invention can form a powder having excellent soft-focus properties.

When the polysaccharide-containing particles contain inorganic particles, at least one spherical particle can be attached to the inorganic particle and/or the coating layer in the polysaccharide-containing particle, and for example, when the inorganic particle is plate-shaped or scale-shaped particle such as mica, it is preferable that at least one spherical particle is attached to both main surfaces of the inorganic particle, directly or via the coating layer. Hereinafter, the particle attached to the inorganic particle means the particle attached to the inorganic particles directly or via the coating layer. The number of spherical particles attached to one inorganic particle is, for example, 1, 2, 3, 5, 9, 10, 15, 20, 30, 31, 40, 50, 60, 70, 80, 90, or 100, and may be within the range between any two of the numerical values exemplified here. The presence or absence of spherical particle attached to the inorganic particle, and the number of spherical particles attached to one inorganic particle can be confirmed by observing the polysaccharide-containing particles with SEM. A part of the spherical particles may not be attached to the inorganic particle, and the polysaccharide-containing particles containing the inorganic particle according to one embodiment of the present invention can also contain the spherical particle that is not attached to the inorganic particles.

The polysaccharide-containing particles according to one embodiment of the present invention can contain 50 parts by mass or more of the total of the polysaccharide, the salt-form polysaccharide, and the neutralizing agent (and inorganic particles as needed) with respect to 100 parts by mass of the polysaccharide-containing particles. The content may be, for example, 50, 60, 70, 80, 90, or 100 parts by mass, and may be within the range between any two of the numerical values exemplified here. The polysaccharide-containing particles according to one embodiment of the present invention can also be composed of a polysaccharide, a salt-form polysaccharide, and a neutralizing agent (and inorganic particles as needed).

The polysaccharide-containing particles according to one embodiment of the present invention may contain known components used in polysaccharide-containing particles, for example, known components used in cosmetics, within a range that does not impair the effects of the present invention. Examples of known components include the components listed as known components that the precursor polysaccharide-containing particles can contain.

3.1 Physical Properties of Polysaccharide-Containing Particles

The polysaccharide-containing particles according to one embodiment of the present invention preferably have a solubility of 50% by mass or less, and more preferably 30% by mass or less, when immersed in water at 25°C for 7 days from the viewpoint of water resistance that can be used as a cosmetic powder. The solubility may be, for example, 0, 5, 10, 15, 20, 25, 30, 35, 40, 45, or 50% by mass, and may be within the range between any two of the numerical values exemplified here. The solubility of the composite powder when immersed in water at 25°C for 7 days can be calculated by immersing the polysaccharide-containing particles in ion-exchanged water at 25°C for 7 days and the following formula, wherein the mass of the polysaccharide-containing particles before immersion is A g, and the mass of the residue after immersion after is B g.

$$[\text{Solubility (\% by mass)}] = (A - B)/A \times 100$$

The solubility of the polysaccharide-containing particles can be controlled by adjusting the type and amount of the polysaccharide, and the production conditions during the production of the polysaccharide-containing particles (the type and amount of the salt-form polysaccharide and the desalting agent to be blended in the dispersion, and the heating temperature and time).

Here, when the polysaccharide-containing particles contain inorganic particles, it is more preferable that the solubility of the residue obtained by removing the inorganic particles from the polysaccharide-containing particles (components containing the polysaccharide and the neutralizing agent) is as described above.

The polysaccharide-containing particles according to one embodiment of the present invention preferably do not exhibit gelation or swelling when 1 g of the polysaccharide-containing particles is immersed in 10 times the amount of ion-exchanged water at 25°C for 7 days. The polysaccharide-containing particles preferably have a lower solubility than the precursor polysaccharide-containing particles before the desalting step. The difference between the solubility of the polysaccharide-containing particles when immersed in water at 25°C for 7 days and the solubility of the precursor polysaccharide-containing particles when immersed in water at 25°C for 7 days is, for example, 5, 10, 15, 20, 25, 30, 35, 40, 45, 50, 55, 60, 65, 70, 75, 80, 85, 90, or 95% by mass, and may be within the range between any two of the numerical values exemplified here.

The polysaccharide-containing particles according to one embodiment of the present invention preferably have a color difference ΔE of the solid content of 0 to 60 when the polysaccharide-containing particles are immersed in water. When the color difference ΔE is within the above range, yellowing and coloring are suppressed, so it is useful as an external preparation for cosmetics and an additive for paints. ΔE can be a value with respect to a standard white in the L*a*b* color space. The color difference ΔE is, for example, 0, 5, 10, 15, 20, 25, 30, 35, 40, 45, 50, 55, or 60, and may be within the range between any two of the numerical values exemplified here. The color difference ΔE of the solid content when the polysaccharide-containing particles are immersed in water can be controlled by adjusting the type and amount of the polysaccharide, and the production conditions at the time of producing the polysaccharide-containing particles (the type and amount of the salt-form polysaccharide and the desalting agent to be blended in the dispersion, and the heating temperature and time), particularly the heating temperature and time.

The polysaccharide-containing particles preferably have biodegradability. Biodegradability means the ability of a material such as polymer to decompose and disappear in the earth's environment, such as in soil or seawater, and/or to decompose and disappear in the living body. As one example, the polysaccharide-containing particles preferably have a BOD degradation degree of 60% or more when exposed to activated sludge for 28 days based on OECD TG 301C. As one example, the polysaccharide-containing particles preferably have a degradation degree of 60% or more or a relative degradation degree with respect to cellulose of 90% or more when buried in soil based on JIS K 6955 (ISO17556). As one example, the polysaccharide-containing particles preferably have a relative degradation degree with respect to cellulose of 60% or more when placed in seawater and sand sediment based on ISO19679. When the polysaccharide-containing particles contain inorganic particles, it is preferable that the biodegradability of the residue (components containing the polysaccharide and the neutralizing agent) obtained by removing the inorganic particles from the polysaccharide-containing particles is as described above.

The polysaccharide-containing particles according to one embodiment of the present invention, particularly when containing inorganic particles, can have a soft focus factor (SFF) of 0.86 or more, and it can be 0.86 to 1.40. The soft focus factor is, for example, 0.86, 0.90, 0.95, 1.00, 1.05, 1.10, 1.15, 1.20, 1.25, 1.3, 1.35, or 1.40, and may be in the range between the two values exemplified herein. The soft focus factor can be measured by applying the polysaccharide-containing particles to a skin material such as Bioskin and using a variable-angle spectrophotometer. The light source is fixed at 45°, the sensor is scanned from 0° to 180° to measure the luminance at each angle, and the ratio of the luminance at two points (the ratio of luminance at 65° to luminance at 135°) can be defined as the soft focus factor. By setting the soft focus factor to the above lower limit or more, it is possible to obtain a cosmetic with suppressed glare. By setting the soft focus factor to the above lower limit or more, it is possible to obtain a bright cosmetic that takes advantage of the high luminance of the inorganic particles themselves, such as mica, which is a extender pigment. The soft focus factor can be adjusted by the type and structure of the components of the polysaccharide-containing particles. In particular, it can be adjusted by controlling the amount of the coating layer on inorganic particles such as mica, and the number and size of the spherical particles.

The polysaccharide-containing particles according to one embodiment of the present invention, particularly when containing inorganic particles, can have an average coefficient of friction (MIU) of 0.75 or less, and it can be 0.40 to 0.75. The average coefficient of friction is, for example, 0.40, 0.45, 0.50, 0.55, 0.60, 0.65, 0.70, or 0.75, and may be in the range between the two values exemplified herein. The average coefficient of friction can be obtained by applying the polysaccharide-containing particles to a skin material such as Bioskin and scanning using a friction feeling tester, and specifically, it can be measured by the method described in the Examples. When the average coefficient of friction is above the lower limit, the cosmetic powder can be maintained in a neat state after application to the skin, and a cosmetic excellent in so-called makeup retention can be obtained. In addition, there is little occurrence of crumbling or falling off during or after molding due to compression, and it is excellent in moldability. When the average coefficient of friction is below the above upper limit, it is possible to obtain a cosmetic that is smooth, has no squeaky feel, and can be applied uniformly. The average coefficient of friction can be adjusted by the type and structure of the components of the polysaccharide-containing particles, and in particular, it can be adjusted by controlling the number and size of the spherical particles attached to inorganic particles such as mica.

The polysaccharide-containing particles according to one embodiment of the present invention preferably have a porosity of 50% or less. The porosity may be, for example, 0, 10, 20, 30, 40, or 50%, and may be in the range between the two values exemplified herein. The polysaccharide-containing particles according to one embodiment of the present invention can exclude porous particles.

By the polysaccharide-containing particles according to one embodiment of the present invention, the polysaccharide-containing particles, which are hardly soluble in water and have little coloring by a mechanism of desalting a salt-form polysaccharide using a desalting agent, can be obtained. According to one embodiment of the present invention, polysaccharide-containing particles that do not contain a crosslinking agent and a crosslinked structure can be obtained. According to one embodiment of the present invention, it is possible to obtain polysaccharide-containing particles that are non-toxic to the ecosystem, have high biodegradability, and reduce the amount of organic solvent used, and it is possible to meet the needs of the industry that wants to create more environmentally friendly products.

The polysaccharide-containing particles according to the present invention have the above characteristics, and are hardly soluble in water and have little coloring, so taking advantage of these characteristics, they can be used for various applications such as external preparations for cosmetics, additives for paints, additives for pharmaceuticals, additives for resin compositions, additives to films for imparting optical or antiblocking properties, and members for processes such as sintering and polishing, and can be used particularly for cosmetics.

Cosmetics can include liquid, gel, solid, and other types of cosmetics, such as foundation, face powder, eye shadow, eye liner, eyebrow, blush, lipstick, nail color and the like.

The polysaccharide-containing particles according to one embodiment of the present invention contain inorganic particles, and it may include inorganic particles sufficiently coated with the coating layer containing the polysaccharide and the salt-form polysaccharide, and further, they may be composite particles in which a sufficient number and size of saccharide-containing spherical particles are attached to the surface of the inorganic particles or the coating layer. According to the polysaccharide-containing particles, a powder with a low average coefficient of friction and a high soft focus factor can be obtained, that is, a cosmetic with excellent slip properties and soft-focus properties can be obtained.

EXAMPLES

Hereinafter, the present invention will be described in more detail based on Examples, but the present invention is not limited to these Examples.

(Example 1)

< Dispersion Preparation step>

Chitosan (manufactured by KOYO CHEMICAL CO.,LTD.), Koyo Chitosan FL-80) and acetic acid were added to water and the mixture was mixed. Urea was further added to the obtained aqueous solution to prepare a dispersion containing a salt-form polysaccharide and a desalting agent with a chitosan concentration of 5 % by mass, an acetic acid concentration of 5 % by mass, and a urea concentration of 0.5 % by mass.

<Spray Drying Step>

Using a two-fluid nozzle type laboratory spray dryer, spray drying was performed with the inlet temperature of the spray dryer set at 200°C to obtain precursor Polysaccharide-Containing Particles 1 containing a desalting agent. When the inlet temperature of the spray dryer was set at 200°C, the outlet temperature was 70 to 120°C.

<Heating Step>

The obtained Precursor Polysaccharide-Containing Particles 1 containing the desalting agent were heated at 120°C for 240 minutes in an air atmosphere to obtain Polysaccharide-Containing Particles 1.
The shape of the obtained Polysaccharide-Containing Particles 1 was observed with SEM, and it was confirmed that spherical particles with a particle diameter of 0.1 to 10 $\mu$m were formed.

(Examples 2 to 18, and Comparative Examples 1 to 8)

Polysaccharide-Containing Particles 2 to 18 were obtained under the dispersion composition and heat treatment conditions shown in Tables 1 to 3.

(Example 19)

< Dispersion Preparation Step>

Mica (average particle size 20 $\mu$m), chitosan (manufactured by KOYO CHEMICAL CO.,LTD., Koyo Chitosan FL-80), and acetic acid were added to water and the mixture was mixed. Urea was further added to the resulting aqueous solution to prepare a dispersion with a mica concentration of 5 % by mass, a chitosan concentration of 5 % by mass, an acetic acid concentration of 5 % by mass, and a urea concentration of 0.5 % by mass.
The spray drying step and the heating step were carried out in the same manner as in Example 1 to obtain Polysaccharide-Containing Particles 19.
The shape of the obtained Polysaccharide-Containing Particles 19 was observed with SEM, and it was confirmed that the mica was coated with a polysaccharide, and that 10 to 30 polysaccharide-containing spherical particles with a particle diameter of 0.1 to 10 $\mu$m were attached to one mica particle. The average coefficient of friction (MIU) was evaluated by the method described later, and it was 0.54. Similarly, the soft focus factor (SFF) was measured by the method described later, and it was 1.05.

(Example 20)

< Dispersion Preparation Step>

Chitosan (manufactured by KOYO CHEMICAL CO.,LTD., Koyo Chitosan FL-80) and acetic acid were added to water and the mixture was mixed. A Dispersion 20, not containing a desalting agent, with a chitosan concentration of 5 % by mass and an acetic acid concentration of 5 % by mass was prepared.

<Spray Drying Step>

Using a two-fluid nozzle type laboratory spray dryer, spray drying was performed with the inlet temperature of the spray dryer set at 200°C to obtain Precursor Polysaccharide-Containing Particles 20 that did not contain a desalting agent. When the inlet temperature of the spray dryer was set at 200°C, the outlet temperature was 70 to 120°C.

<Desalting Step>

10 g of a 30% aqueous ammonia solution (containing 3 g of ammonia), which is a nitrogen-containing compound, 6 g of water, and 2 g of methanol were mixed to prepare a solution containing a desalting agent. 5 g of the obtained precursor polysaccharide-containing particles were added to an aqueous alcohol solution prepared by mixing 5 g of water and 5 g of methanol, and the mixture was stirred. While maintaining the dispersed state by stirring, the above solution containing the desalting agent was slowly added. After adding the desalting agent solution and stirring for 3 hours, suction filtration was performed through a mesh, and the obtained cake was washed with 100 g of tap water and suction filtered. After sufficiently removing water by suction filtration, it was dried in a dryer at 50°C for 5 hours to obtain Polysaccharide-Containing Particles 20. All of the above operations were performed at room temperature (23°C).

(Examples 21 to 27)

Polysaccharide-containing particles 21 to 27 were obtained in the same manner as in Example 20, except that the composition of the dispersant was as shown in Table 4, and the type of nitrogen-containing compound was as shown in Table 4. The amount of each desalting agent in the desalting step in Table 4 indicates the amount with respect to 5 parts by mass of the precursor polysaccharide-containing particles.

(Example 28)

< Dispersion Preparation Step and Spray Drying Step>

A dispersion 28 was obtained in the same manner as in Example 1, except that the composition of the dispersion was as shown in Table 4. Also, in the same manner as in Example 1, a spray drying step was performed to obtain Precursor Polysaccharide-Containing Particles 28.

<Desalting step>

5 g of the obtained precursor polysaccharide-containing particles were added to an aqueous alcohol solution prepared by mixing 5 g of water and 10 g of isopropyl alcohol, and the mixture was stirred. While maintaining the dispersed state by stirring, the solution containing the above desalting agent was slowly added. After adding the desalting agent solution and stirring for 3 hours, suction filtration was performed through a mesh, and the obtained cake was washed with 100 g of tap water and suction filtered. After sufficiently removing water by suction filtration, it was dried in a dryer at 50°C for 5 hours to obtain Polysaccharide-Containing Particles 28. All of the above operations were performed at room temperature (23°C).

<Examples 29 and 30>

Polysaccharide-Containing Particles 29 and 30 were obtained in the same manner as in Example 28, except that methanol and ethanol were used instead of isopropyl alcohol, and the type and amount of the desalting agent used in the desalting step were as shown in Table 4.

The raw materials used to produce the dispersion are shown below.

Natural mica: average particle size 20 $\mu$m,
Chitosan: manufactured by KOYO CHEMICAL CO.,LTD., Koyo Chitosan FL-80, degree of deacetylation 75% or more
CMC-NH$_4$ (carboxymethyl cellulose ammonium): NICHIRIN CHEMICAL INDUSTRIES, LTD., Kiccolate NA-3L

(Evaluation of Polysaccharide-Containing Particles)

<Color difference (∆E) of Particles with Respect to Standard White When Immersed in Water >

The obtained polysaccharide-containing particles were immersed in ion-exchanged water, and the color tone of the solid content was measured in the L*a*b* color space using a Color Reader CR-13 (manufactured by KONICA MINOLTA, INC.), and the color difference ∆E with respect to the standard white (L=100, a=b=0) was calculated and evaluated according to the following evaluation criteria. The results are shown in Tables 1 to 4.

<Solubility>

1 g of the polysaccharide-containing particles was immersed in 10 times its mass of ion-exchanged water at 25°C for 7 days, and then the mixture was suction filtered through a glass fiber filter, and the residue captured by the filter was dried in a dryer at 100°C for 2 hours, and the mass of the residue was calculated. The mass of the sample before immersion was defined as A g, and the mass of the residue was defined as B g, and the solubility of each sample was calculated by the following formula and evaluated according to the following evaluation criteria. The results are shown in Tables 1 to 4.

$$[\text{Solubility (\% by mass)}] = (A - B)/A \times 100$$

A: 30% or less
B: more than 30% and 50% or less
C: more than 50%

<State of Particles When Immersed in Water >

1 g of the polysaccharide-containing particles was immersed in 10 times the amount of ion-exchanged water at 25°C for 7 days, and the state was observed.
C: The polysaccharide-containing particles absorbed water and/or dissolved in water and were in a gelled state.
B: The polysaccharide-containing particles were precipitated in water, and the particles absorbed at least some water, swelled, and increased in volume.
A: The polysaccharide-containing particles were precipitated in water, and no swelling of the particles was observed.

<Average Coefficient of Friction (MIU)>

The obtained polysaccharide-containing particles 19 were applied to Bioskin (manufactured by Beaulax Co.,Ltd) at 1 mg/cm$^2$, and the friction coefficient was measured by scanning 30 mm at a speed of 1 mm/s with a load of 50 N using a friction tester KES, and the average value of the central 20 mm was taken as the average coefficient of friction (MIU).
<Soft focus factor (SFF)>
The obtained polysaccharide-containing particles 19 were applied to Bioskin (manufactured by Beaulax Co.,Ltd) at a 10 mg/cm$^2$, and the soft focus factor was evaluated using a variable-angle photometer (GC-5000L manufactured by NIPPON DENSHOKU INDUSTRIES CO., LTD.). Specifically, the light source was fixed at a position of 45°, the sensor was scanned from 0° to 180° with respect to the Bioskin surface coated with the composite powder, the luminance at each angle was measured, and the ratio of the luminance at two points (the ratio of luminance at 65° to luminance at 135°) was defined as the soft focus factor.

[Table 1]

| table 1 | | | unit | Example | | | | | | | | |
|---|---|---|---|---|---|---|---|---|---|---|---|---|
| | | | | 1 | 2 | 3 | 4 | 5 | 6 | 7 | 8 | 9 |
| dispersion formulation | polysaccharide | chitosan | % by mass | 5 | 5 | 5 | 5 | 5 | 5 | 5 | 5 | 5 |
| | salt-forming agent | acetic acid | % by mass | 5 | | 5 | 5 | 5 | 5 | 5 | 5 | 5 |
| | | lactic acid | % by mass | | 5 | | | | | | | |
| | salt-form polysaccharide | CMC-NH$_4$ | % by mass | | | | | | | | | |
| | desalting agent | nitrogen-containing compound | urea | % by mass | 0.50 | 0.50 | 0.50 | 0.50 | 0.50 | | | | |
| | | | methylurea | % by mass | | | | | | 0.50 | | | |
| | | | butylurea | % by mass | | | | | | | 0.50 | | |
| | | | tetramethylurea | % by mass | | | | | | | | 0.50 | |
| | | | niacin | % by mass | | | | | | | | | 0.50 |
| | | | niacinamide | % by mass | | | | | | | | | |
| | | | amylamine | % by mass | | | | | | | | | |
| | | | isopropanolamine | % by mass | | | | | | | | | |
| | | | N-ethylpiperidine | % by mass | | | | | | | | | |
| | | | polyethylenediamine | % by mass | | | | | | | | | |

(continued)

| table 1 | | | | unit | Example | | | | | | | | |
|---|---|---|---|---|---|---|---|---|---|---|---|---|---|
| | | | | | 1 | 2 | 3 | 4 | 5 | 6 | 7 | 8 | 9 |
| | | carboxylic acid | acetic acid | % by mass | | | | | | | | | |
| | | | lactic acid | % by mass | | | | | | | | | |
| heat treatment condition | tempreture | | | °C | 120 | 120 | 160 | 180 | 180 | 120 | 120 | 120 | 120 |
| | time | | | minuite | 240 | 240 | 60 | 10 | 30 | 240 | 240 | 240 | 240 |
| evaluation of polysaccharide-containing particles | color difference of particles when immersed in water with respect to standard white (ΔE) | | | | 23.7 | 39.8 | 27.7 | 28.8 | 31.8 | 58.1 | 59.0 | 51.8 | 59.5 |
| | solubility | | | | A | A | A | A | A | A | A | A | A |
| | state of particles when immersed | | | | A | A | A | A | A | A | A | A | A |

[Table 2]

| table 2 | | unit | Example | | | | | | | | |
|---|---|---|---|---|---|---|---|---|---|---|---|
| | | | 10 | 11 | 12 | 13 | 14 | 15 | 16 | 17 | 18 |
| dispersion formulation | polysaccharide — chitosan | % by mass | 5 | 5 | 5 | 5 | 5 | | | | |
| | salt-forming agent — acetic acid | % by mass | 5 | 5 | 5 | 5 | | | | | |
| | salt-forming agent — lactic acid | % by mass | | | | | 5 | | | | |
| | salt-form polysaccharide — CMC-NH$_4$ | % by mass | | | | | | 2.5 | 2.5 | 2.5 | 2.5 |
| | nitrogen-containing compound — urea | % by mass | | | | | | | | | |
| | nitrogen-containing compound — methylurea | % by mass | | | | | | | | | |
| | nitrogen-containing compound — butylurea | % by mass | | | | | | | | | |
| | nitrogen-containing compound — tetramethylurea | % by mass | | | | | | | | | |
| | nitrogen-containing compound — niacin | % by mass | | | | | | | | | |
| | nitrogen-containing compound — niacinamide | % by mass | 0.50 | | | | | | | | |
| | desalting agent — amylamine | % by mass | | 0.50 | | | | | | | |
| | desalting agent — isopropanolamine | % by mass | | | 0.50 | | | | | | |
| | desalting agent — N-ethylpiperidine | % by mass | | | | 0.50 | | | | | |
| | desalting agent — polyethylenediamine | % by mass | | | | | 0.50 | | | | |

| table 2 | | | | unit | Example | | | | | | | | |
|---|---|---|---|---|---|---|---|---|---|---|---|---|---|
| | | | | | 10 | 11 | 12 | 13 | 14 | 15 | 16 | 17 | 18 |
| | | carboxylic acid | acetic acid | % by mass | | | | | | 0.25 | | 0.25 | 0.25 |
| | | | lactic acid | % by mass | | | | | | | 0.25 | | |
| heat treatment condition | tempreture | | | °C | 120 | 120 | 120 | 120 | 120 | 120 | 120 | 100 | 160 |
| | time | | | minuite | 240 | 240 | 240 | 240 | 240 | 30 | 30 | 240 | 10 |
| evaluation of poly-saccharide-con-taining particles | color difference of particles when immersed in water with respect to standard white (ΔE) | | | | 58.0 | 59.0 | 43.0 | 34.3 | 43.5 | 27.7 | 27.8 | 26.1 | 29.5 |
| | solubility | | | | A | A | A | A | A | A | A | A | A |
| | state of particles when immersed | | | | A | A | A | A | A | A | A | A | A |

EP 4 613 797 A1

[Table 3]

| table 3 | | | unit | Comparative Example | | | | | | | |
|---|---|---|---|---|---|---|---|---|---|---|---|
| | | | | 1 | 2 | 3 | 4 | 5 | 6 | 7 | 8 |
| dispersion formulation | polysaccharide | chitosan | % by mass | 5 | 5 | 5 | 5 | | | | 5 |
| | salt-forming agent | acetic acid | % by mass | 5 | 5 | 5 | 5 | | | | 5 |
| | salt-form polysaccharide | CMC-NH$_4$ | % by mass | | | | | 2.5 | 2.5 | 2.5 | |
| | desalting agent | nitrogen-containing compound | urea | % by mass | | | | | | | |
| | | carboxylic acid | acetic acid | % by mass | | | | | | | |
| heat treatment condition | tempreture | | °C | 120 | 160 | 180 | 180 | 120 | 120 | 140 | - |
| | time | | minuite | 240 | 60 | 10 | 30 | 30 | 60 | 60 | - |
| evaluation of polysaccharide-containing particles | color difference of particles when immersed in water with respect to standard white (ΔE) | | | 67.4 | 64.5 | 74.4 | 73.9 | 21.4 | 25.3 | 60.3 | 15.4 |
| | solubility | | | C | A | B | A | C | B | A | C |
| | state of particles when immersed | | | C | A | B | A | C | B | A | C |

[Table 4]

| table 4 | | | unit | Example | | | | | | | | | | |
|---|---|---|---|---|---|---|---|---|---|---|---|---|---|---|
| | | | | 20 | 21 | 22 | 23 | 24 | 25 | 26 | 27 | 28 | 29 | 30 |
| dispersion formulation | polysaccharide | chitosan | % by mass | 5 | 5 | 5 | 5 | 5 | 5 | 5 | | 5 | 5 | 5 |
| | salt-forming agent | acetic acid | % by mass | 5 | | 5 | 5 | 5 | 5 | 5 | | 5 | 5 | 5 |
| | | lactic acid | % by mass | | 5 | | | | | | | | | |
| | salt-form polysaccharide | CMC-NH$_4$ | % by mass | | | | | | | | 5 | | | |
| desalting step | desalting agent | ammonia (nitrogen-containing compound) | parts by mass | 3.00 | 3.00 | | | | | | | | | |
| | | sodium hydroxide | parts by mass | | | 3.00 | | | | | | | | |
| | | tetrahydroxy propylethylenediamine | parts by mass | | | | 3.00 | | | | | | | |
| | | 1-amino-3-undecanoxy-propane | parts by mass | | | | | 3.00 | | | | | | |
| | | dimethylaminoethanol | parts by mass | | | | | | 3.00 | | | | | |
| | | polyethylenediamine | parts by mass | | | | | | | 3.00 | | | | |
| | alcohol | methanol | parts by mass | 7.00 | 7.00 | 7.00 | 7.00 | 7.00 | 7.00 | 7.00 | 7.00 | | 10.00 | |
| | | ethanol | | | | | | | | | | | | 10.00 |
| | | isopropyl alcohol | parts by mass | | | | | | | | | 10.00 | | |
| | acid | lactic acid | parts by mass | | | | | | | | 3.00 | | | |
| heat treatment condition | tempreture | | °C | - | - | - | - | - | - | - | - | - | - | - |
| | time | | minuite | - | - | - | - | - | - | - | - | - | - | - |

EP 4 613 797 A1

23

(continued)

table 4

| evaluation of polysaccharide-containing parti-cles | | unit | Example | | | | | | | | | | | |
|---|---|---|---|---|---|---|---|---|---|---|---|---|---|---|
| | | | 20 | 21 | 22 | 23 | 24 | 25 | 26 | 27 | 28 | 29 | 30 |
| color difference of particles when immersed in water with respect to standard white (ΔE) | | | 16.0 | 18.5 | 16.4 | 16.6 | 17.3 | 17.7 | 18.9 | 15.6 | 15.8 | 15.6 | 15.6 |
| solubility | | | A | A | A | A | A | A | A | A | A | B | B |
| state of particles when immersed | | | A | A | A | A | A | A | A | A | A | B | B |

**Claims**

1. A method for producing polysaccharide-containing particles comprising:

   a spray drying step; and
   a desalting step,
   wherein:

   in the spray drying step, a dispersion containing a salt-form polysaccharide is spray dried to obtain precursor polysaccharide-containing particles, and
   in the desalting step, a desalting agent is brought into contact with the salt-form polysaccharide contained in the precursor polysaccharide-containing particles, and the salt-form polysaccharide is desalted with the desalting agent to obtain polysaccharide-containing particles.

2. The method for producing polysaccharide-containing particles of Claim 1, wherein the desalting step includes a heat treatment step, and in the heat treatment step, the precursor polysaccharide-containing particles are heated in a state where the desalting agent is in contact with the salt-form polysaccharide, and the salt-form polysaccharide is desalted with the desalting agent to obtain polysaccharide-containing particles.

3. The method for producing polysaccharide-containing particles of Claim 1, wherein the dispersion further comprises inorganic particles.

4. The method for producing polysaccharide-containing particles of Claim 1 or 2, wherein the desalting agent is a nitrogen-containing compound, a carboxylic acid, or an alcohol.

5. The method for producing polysaccharide-containing particles of Claim 4, wherein the nitrogen-containing compound is at least one selected from a group consisting of carbamide, amine, acid amide, and a nitrogen-containing heterocyclic compound.

6. The method for producing polysaccharide-containing particles of Claim 1 or 2, wherein the polysaccharide is at least one selected from a group consisting of chitosan, cellulose, and derivatives thereof.

7. The method for producing polysaccharide-containing particles of Claim 1 or 2, wherein a color difference $\Delta E$ of solid content when polysaccharide-containing particles are immersed in water is 0 to 60, and the color difference $\Delta E$ is a value with respect to a standard white in L*a*b* color space.

8. The method for producing polysaccharide-containing particles of Claim 2, wherein, in the heating step, heating treatment is performed at 100°C or higher and lower than 200°C.

9. Precursor polysaccharide-containing particles comprising a salt-form polysaccharide and a desalting agent capable of desalting the salt-form polysaccharide.

10. Polysaccharide-containing particles comprising a polysaccharide and a neutralizing agent, wherein the neutralizing agent is capable of neutralizing an acid or a base capable of forming a salt with the polysaccharide.

11. Polysaccharide-containing particles of Claim 10, wherein a solubility when immersed in water at 25°C for 7 days is 50% by mass or less.

12. Polysaccharide-containing particles of Claim 10 or 11, further comprising inorganic particles.

## INTERNATIONAL SEARCH REPORT

| | International application No. |
|---|---|
| | **PCT/JP2023/039305** |

**A.    CLASSIFICATION OF SUBJECT MATTER**

*C08J 3/12*(2006.01)i; *A61K 8/73*(2006.01)i; *C08B 11/04*(2006.01)i; *C08B 37/08*(2006.01)i
FI:    C08J3/12 101; A61K8/73; C08B11/04; C08B37/08; C08J3/12 CEP

According to International Patent Classification (IPC) or to both national classification and IPC

**B.    FIELDS SEARCHED**

Minimum documentation searched (classification system followed by classification symbols)

C08J3/00-3/28; 99/00; A61K8/00-8/99; C08B1/00-37/18

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | JP 63-210101 A (FUJI SPINNING CO., LTD.) 31 August 1988 (1988-08-31) | 1-2, 4-6, 8-11 |
| | claim 1, column 2, lines 3-6, column 4, line 14 to column 8, line 6 | |
| Y | | 3, 12 |
| A | | 7 |
| X | JP 2017-186489 A (FUJIKURA KASEI CO., LTD.) 12 October 2017 (2017-10-12) | 9-12 |
| | claim 1, 4, paragraphs [0004], [0007]-[0020], [0033]-[0034] | |
| A | | 1-8 |
| X | JP 8-239311 A (SEKISUI PLASTICS CO., LTD.) 17 September 1996 (1996-09-17) | 9, 10, 12 |
| | claims 1-2, paragraphs [0008], [0011]-[0014], [0019], [0028]-[0033], [0036]-[0037] | |
| A | | 1-8, 11 |

☑ Further documents are listed in the continuation of Box C.        ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **14 February 2024** | **27 February 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**EP 4 613 797 A1**

**INTERNATIONAL SEARCH REPORT**

| International application No. |
|---|
| **PCT/JP2023/039305** |

### C. DOCUMENTS CONSIDERED TO BE RELEVANT

| Category* | Citation of document, with indication, where appropriate, of the relevant passages | Relevant to claim No. |
|---|---|---|
| X | JP 64-66204 A (FUJI SPINNING CO., LTD.) 13 March 1989 (1989-03-13) claim 1, column 2, line 2 to column 3, line 6 | 9–12 |
| Y |  | 3, 12 |
| A |  | 1-2, 4-8 |
| A | JP 2022-135891 A (SEKISUI PLASTICS CO., LTD.) 15 September 2022 (2022-09-15) claims 1, 5, 9-10, paragraphs [0001], [0029]-[0030], [0039]-[0056] | 1-12 |
| A | JP 2022-94097 A (SEKISUI PLASTICS CO., LTD.) 24 June 2022 (2022-06-24) claims 1-8, paragraphs [0001], [0016]-[0042] | 1-12 |
| A | JP 2022-46073 A (SEKISUI PLASTICS CO., LTD.) 23 March 2022 (2022-03-23) claims 1-4, 6-9, paragraphs [0001], [0014]-[0019], [0032], [0040], [0046]-[0060] | 1-12 |

Form PCT/ISA/210 (second sheet) (July 2022)

27

**INTERNATIONAL SEARCH REPORT**

International application No.

**PCT/JP2023/039305**

**Box No. III    Observations where unity of invention is lacking (Continuation of item 3 of first sheet)**

This International Searching Authority found multiple inventions in this international application, as follows:

(Invention 1) Claims 1-8

The technical features of the inventions in claims 1, 2, 4-6, and 8 do not make a contribution over the prior art in light of the disclosure of JP 63-210101 A, etc., and thus these inventions do not have a special technical feature.

Subsequently, in the invention in claim 3, found was the special technical feature of the "production method set forth in claim 1, wherein the dispersion liquid further comprises inorganic particles."

In addition, in the invention in claim 7, found was the special technical feature of the "production method set forth in claim 1 or claim 2, wherein when the polysaccharide-containing particles are immersed in water, the color difference ΔE of the solid content is 0-60, and the color difference ΔE is a value with respect to a reference white color in an L✳a✳b✳ color space."

Therefore, the inventions in claims 1 to 8, for which the presence or absence of special technical features has been examined so far are classified as invention 1.


(Invention 2) Claim 9

The invention in claims 9 shares, with the invention in claims 3 and 7, the common technical feature of "polysaccharides in salt state," "desalting agents," and "precursor polysaccharide-containing particles."

However, mere "polysaccharides in salt state," "desalting agents," and "precursor polysaccharide-containing particles" do not make a contribution over the prior art in light of the disclosure of JP 63-210101 A, etc., and thus said technical features cannot be said to be a special technical feature.

In addition, there are no other same or corresponding special technical features between these inventions.

Therefore, the invention in claim 9 cannot be classified as invention 1, and is thus classified as invention 2.


(Invention 3) Claims 10-12

The invention in claims 10-12 shares, with the invention in claims 3 and 7, the common technical feature of "polysaccharides," and "polysaccharide-containing particles."

However, mere "polysaccharides," and "polysaccharide-containing particles" do not make a contribution over the prior art in light of the disclosure of JP 63-210101 A, etc., and thus said technical features cannot be said to be a special technical feature.

In addition, the invention in claim 11 including all the specifying matters of the invention in claims 10-12 shares, with the invention in claim 3, the common technical feature of "polysaccharides," "polysaccharide-containing particles," and "inorganic particles."

However, mere "polysaccharides," "polysaccharide-containing particles," and "inorganic particles" do not make a contribution over the prior art in light of the disclosure of JP 2017-186489 A, etc., and thus said technical features cannot be said to be a special technical feature.

In addition, there are no other same or corresponding special technical features between these inventions.

In addition, there are no other same or corresponding special technical features between the invention in claims 10-12 and the invention classified as invention 2 in light of the descriptions with respect to invention 2.

Therefore, claims 10-12 cannot be classified as either invention 1 or invention 2, and are thus classified as invention 3.

Form PCT/ISA/210 (continuation of first sheet) (July 2022)

## INTERNATIONAL SEARCH REPORT

| | International application No. |
|---|---|
| | **PCT/JP2023/039305** |

**Box No. III      Observations where unity of invention is lacking (Continuation of item 3 of first sheet)**

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

## INTERNATIONAL SEARCH REPORT
### Information on patent family members

International application No.

**PCT/JP2023/039305**

| Patent document cited in search report | | | Publication date (day/month/year) | Patent family member(s) | Publication date (day/month/year) |
|---|---|---|---|---|---|
| JP | 63-210101 | A | 31 August 1988 | (Family: none) | |
| JP | 2017-186489 | A | 12 October 2017 | (Family: none) | |
| JP | 8-239311 | A | 17 September 1996 | (Family: none) | |
| JP | 64-66204 | A | 13 March 1989 | (Family: none) | |
| JP | 2022-135891 | A | 15 September 2022 | (Family: none) | |
| JP | 2022-94097 | A | 24 June 2022 | (Family: none) | |
| JP | 2022-46073 | A | 23 March 2022 | (Family: none) | |

Form PCT/ISA/210 (patent family annex) (July 2022)

**EP 4 613 797 A1**

**Patent documents cited in the description**

- JP H827277 A

- JP 2022094097 A